# EUROPÄISCHE PATENTSCHRIFT

(11) **EP 3 147 707 B1**
(45) Veröffentlichungstag und Bekanntmachung des Hinweises auf die Patenterteilung: **24.04.2019**
(21) Anmeldenummer: 15186958.3
(22) Anmeldetag: 25.09.2015
(51) Int. Cl.: G03F 7/00, A61K 6/02, A61K 6/027, G03F 7/004, G03F 7/027

(54) **KERAMIK- UND GLASKERAMIKSCHLICKER FÜR DIE STEREOLITHOGRAPHY**
CERAMIC AND GLASS CERAMIC SLURRY FOR STEREO LITHOGRAPHY
BARBOTINE EN CERAMIQUE OU EN VITROCERAMIQUE POUR LA STEREOLITHOGRAPHIE

(43) Veröffentlichungstag der Anmeldung: 29.03.2017
(73) Patentinhaber: Ivoclar Vivadent AG, 9494 Schaan (LI)
(72) Erfinder: BONDERER, Lorenz Josef, 7320 Sargans (CH); LAUBERSHEIMER, Jürgen, 9470 Buchs (CH)
(74) Vertreter: Uexküll & Stolberg

(56) Entgegenhaltungen:
- EP-A1- 2 151 214
- EP-A1- 2 636 511
- EP-A2- 0 130 031
- EP-A2- 0 186 163
- WO-A1-2015/111366
- US-A1- 2003 109 588
- US-A1- 2007 249 770
- US-A1- 2008 103 226

## Beschreibung

Die vorliegende Erfindung betrifft Schlicker für die stereolithographische Herstellung von Keramik- oder Glaskeramikformteilen, wie dentalen Inlays, Onlays, Veneers, Kronen, Brücken und Gerüsten.

Unter dem Begriff "Rapid Prototyping" (RP), werden generative Fertigungsverfahren zusammengefasst, bei denen aus computergestützten Konstruktionsdaten (CAD-Daten) 3-dimensionale Modelle oder Bauteile hergestellt werden (A. Gebhardt, Vision of Rapid Prototyping, Ber. DGK 83 (2006) 7-12). Dabei handelt es sich um Verfahren, wie z.B. Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF) und Direct Ceramic Jet Printing (DCJP), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77ff.). Bei der Stereolithographie handelt es sich um RP-Verfahren (A. Beil, Fertigung von Mikro-Bauteilen mittels Stereolithographie, Düsseldorf 2002, VDI-Verlag 3 ff.), bei denen ein Formteil auf der Basis von CAD-Daten schichtweise aus einem flüssigen und härtbaren Monomerharz aufgebaut wird.

Stereolithographische Verfahren zur Herstellung von dentalen Formkörpern, wie Inlays, Kronen oder Brücken, sind vor allem bei keramischen Werkstoffen von Vorteil, weil damit eine deutliche Vereinfachung der im zahntechnischen Labor mit erheblichen manuellen Aufwand betriebenen Abform- und Gießprozesse bzw. der Fräs- und Schleifoperationen möglich ist und gleichzeitig der bei nicht-generativen Verfahren auftretende große Materialverlust vermieden werden kann. Da heutzutage eine vollständige digitale Prozesskette etabliert ist, lassen sich die klassischen Verfahrensschritte zur Herstellung von z.B. mehrgliedrigen Brückengerüsten (Ausrichten im Artikulator, Wachsmodulation, Einbetten und Guss) durch die Digitalisierung des Modells, virtuelle Konstruktion des dentalen Formkörpers und dessen generative stereolithographische Fertigung ersetzen.

Bei der stereolithographischen Herstellung von keramischen Formteilen wird zunächst ein keramischer Grünling durch schichtweise Strahlungshärtung eines fließfähigen keramischen Schlickers hergestellt, der dann nach der Entbinderung zu einem dichten keramischen Formkörper gesintert wird. Der Grünling wird auch Grünkörper genannt. Als Entbinderung wird das Austreiben des Bindemittels bezeichnet. Hierbei wird das verwendete Bindemittel üblicherweise durch Erhitzen des Grünlings auf eine Temperatur von ca. 80°C bis 600°C entfernt. Wesentlich ist, dass die Bildung von Rissen und Deformationen weitestgehend vermieden wird. Durch die Entbinderung wird aus dem Grünling der sogenannte Weißling. Beim Entbindern wird das Bindemittel durch thermische und thermo-chemische Prozesse zu flüchtigen Komponenten abgebaut.

Das Entbindern ist ein kritischer Schritt des Verfahrens. Hier ist die Gefahr groß, dass das Bauteil durch Gase, die bei der Zersetzung der organischen Matrix entstehen, und den durch diese ausgeübten Druck beschädigt wird. Besonders groß ist die Gefahr, dass kleine Defekte zwischen den einzelnen Bauschichten beim Entbindern zu Rissen oder gar zur vollständigen Zerstörung des Bauteils führen. Dieses Risiko kann dadurch reduziert werden, dass die Entbinderungsdauer erhöht wird, was jedoch die Prozesszeit stark verlängert.

Die Sinterung des Weißlings erfolgt im Sinterofen beim Hochtemperaturbrand. Dabei kommt es zur Verdichtung und Verfestigung des fein verteilten Keramikpulvers durch Temperatureinwirkung unterhalb der Schmelztemperatur der Hauptkomponenten, wodurch das poröse Bauteil kleiner wird und dessen Festigkeit zunimmt.

Die US 5,496,682 offenbart lichthärtbare Zusammensetzungen für die Herstellung dreidimensionaler Körper durch Stereolithographie, die 40 bis 70 Vol.-% Keramik- oder Metallpartikel, 10 bis 35 Gew.-% Monomer, 1 bis 10 Gew.-% Photoinitiator, 1 bis 10 Gew.-% Dispergiermittel und vorzugsweise auch Lösungsmittel, Weichmacher und Kopplungsmittel enthalten.

Die US 6,117,612 beschreibt Harze für die stereolithographische Herstellung von gesinterten Keramik- oder Metallteilen. Die Harze haben eine Viskosität von weniger als 3000 mPa·s. Zur ihrer Herstellung werden Monomere mit geringer Viskosität eingesetzt, vorzugsweise in wässriger Lösung. Durch die Verwendung von Dispersionsmitteln soll ein hoher Feststoffgehalt bei geringer Viskosität erzielt werden.

Die DE 10 2005 058 116 A1 offenbart Suspensionen zur stereolithographischen Herstellung von keramischen Implantaten auf die in der US 6,117,612 beschriebene Weise, die keine Verdünnungsmittel wie Wasser oder organische Lösungsmittel enthalten, da diese durch lokales Verdampfen beim Energieeintrag die Viskosität erhöhen sollen. Die Viskosität der Suspension wird durch Variation der Konzentration eines Dispergators auf weniger als 20 Pa·s eingestellt. Als Dispergator werden Alkylammoniumsalze von Copolymeren mit sauren Gruppen eingesetzt, wobei diese auch auf die Partikel des keramischen Pulvers geschichtet werden können.

In der US 2005/0090575 A1 werden Methoden und Zusammensetzungen für die stereolithographische Herstellung von keramischen Bauteilen beschrieben. Es wird angegeben, dass mit den aus US 5,496,682 bekannten flüssigen Materialien hergestellte Formteile weich sind und daher einen zusätzlichen Härtungsschritt erfordern, um Verformungen beim Brennen zu vermeiden, während aus pastenförmigen Materialien gewonnene Formkörper bei der Entbinderung innere Spannungen aufbauen, die beim Sintern zu Rissen führen. Zur Vermeidung dieser Probleme werden Weichmacher eingesetzt und die Menge des Keramikpulvers so gewählt, dass die Viskosität der Zusammensetzungen mindestens 10.000 Pa·s beträgt.

Aus der EP 2 233 449 A1 sind Schlicker zur Herstellung von Keramikformteilen durch Hot-Melt-Inkjet-Druckverfahren bekannt, die Keramikpartikel, Wachs und mindestens ein radikalisch polymerisierbares Wachs enthalten und die Grünkörper ergeben, die sich ohne Rissbildung entbindern lassen. Nachteilig an diesen Schlickern ist, dass sie im flüssigen Zustand bei längerem Stehen zur Entmischung neigen. Dies ist bei Hot-Melt-Inkjet-Verfahren unkritisch, weil die Schlicker nur während des Druckvorgangs, d.h. für einen relativ kurzen Zeitraum in flüssiger Form vorliegen. Bei stereolithographischen Verfahren müssen die Schlicker jedoch über längere Zeiträume in flüssiger Form stabil sein, d.h. insbesondere, dass sich die im Schlicker dispergierten Partikel nicht vorzeitig absetzen dürfen, was im Hinblick auf den angestrebten möglichst hohen Volumenanteil der keramischen Partikel im Schlicker ein besonderes Problem darstellt. EP2151214 A1 offenbart Schlicker, die ein Dispergiermittel enthalten können. Als Dispergiermittel werden nichtionische Polymeren genannt.

US 2007/249770, EP0130031 A2, US2003/109588 und WO2015/111366 offenbaren Zusammensetzungen, u.a.,Schlicker, die ein nichtionisches Tensid enthalten können.

Außerdem sollen Schlicker für stereolithographische Verfahren eine hohe Reaktivität aufweisen und so kurze Belichtungs- und Prozesszeiten ermöglichen. Sie müssen eine gute Grünkörperfestigkeit und gute Formstabilität, hohe Genauigkeit und Präzision nach dem Entbindern, Sintern und abschießenden Reinigen der Körper gewährleisten.

Die bekannten Schlicker sind hinsichtlich der oben genannten Anforderungen für stereolithographische Verfahren nicht optimal. Der Erfindung liegt daher die Aufgabe zugrunde, verbesserte lichthärtende Schlicker zur stereolithographischen Herstellung von Keramik- und Glaskeramikformteilen bereitzustellen, die den obigen Anforderungen genügen und die die genannten Nachteile nicht aufweisen. Die Schlicker sollen insbesondere im flüssigen Zustand sedimentationsstabil sein. Sie sollen Grünlinge mit hoher Gründichte und Festigkeit ergeben, die sich ohne Verformung, Riss- oder Spannungsbildung entbindern lassen. Die Grünlinge sollen beim Sintern zu hochfesten Keramiken führen, die für dentale Zwecke geeignet sind. Erfindungsgemäß wird diese Aufgabe durch Schlicker gelöst, die
(a) mindestens ein radikalisch polymerisierbares Monomer,
(b) mindestens einen Photoinitiator,
(c) Keramik- und/oder Glaskeramikpartikel und
(d) mindestens ein nichtionisches Tensid enthalten.

Nichtionische Tenside sind Stoffe mit grenzflächenaktiven Eigenschaften, die in wässrigen Medien keine Ionen bilden. Es handelt sich um Moleküle, die einen hydrophoben und einen hydrophilen Teil aufweisen. Durch Wahl der Länge und Art der hydrophoben und hydrophilen Teile kann die Gesamt-Hydrophobizität der Moleküle eingestellt werden.

Das nichtionische Tensid (d) ist ein Tensid mit einem Schmelzpunkt von 30°C bis 120°C, bevorzugt 40°C bis 100°C, besonders bevorzugt 40°C bis 60°C.

Erfindungsgemäß sind nichtionische Tenside mit einem HLB-Wert im Bereich von 3 bis 16, insbesondere 4 bis 13 und ganz besonders von 4 bis 10 bevorzugt. Der HLB-Wert wird nach der Methode von Griffin bestimmt.

Bevorzugte nichtionische Tenside (d) sind die Ethoxylate von Fettalkoholen, Oxoalkoholen oder Fettsäuren, Fettsäureester von Zuckern und hydrierten Zuckern, Alkylglycoside sowie Blockpolymere des Ethylen- und Propylenoxids, insbesondere kurzkettige Block-Co-Oligomere.

Besonders bevorzugt sind Fettsäuresester von hydrierten Zuckern, insbesondere solche mit der Formel R'-CO-O-Zucker, wobei R' ein verzweigter oder bevorzugt gerader Alkylrest mit 10 bis 25 Kohlenstoffatomen, vorzugsweise 12 bis 22 Kohlenstoffatomen ist. Besonders bevorzugt sind gerade Alkylreste mit 16 bis 22 Kohlenstoffatomen. "Zucker" steht für einen hydrierten Zuckerrest, der vorzugsweise 1- bis 5-fach ethoxyliert ist. Ganz besonders bevorzugt sind Fettsäureester des Sorbitols, insbesondere Sorbitanstearate wie z.B. Sorbitanmonostearat (CAS 1338-41-6).

Eine weitere besonders bevorzugte Gruppe von Tensiden sind Ethoxylate von Fettsäuren, insbesondere solche mit der allgemeinen Formel R"-(CO)-(OCH₂CH₂)ₘ-OH, in der R" ein verzweigter oder bevorzugt gerader Alkylrest mit 10 bis 25 Kohlenstoffatomen, vorzugsweise 12 bis 22 Kohlenstoffatomen ist. Besonders bevorzugt sind gerade Alkylreste mit 16 bis 22 Kohlenstoffatomen. m ist eine ganze Zahl von 2 bis 20, bevorzugt 2 bis 10 und besonders bevorzugt 2 bis 6.

Erfindungsgemäß ganz besonders bevorzugte Tenside sind Ethoxylate von Fettalkoholen, insbesondere Polyalkylenglycolether mit der allgemeinen Formel R-(OCH₂CH₂)ₙ-OH, in der R ein Alkylrest mit 10 bis 20 Kohlenstoffatomen und n eine ganze Zahl von 2 bis 25 ist. R kann ein verzweigter oder bevorzugt gerader Alkylrest sein, wobei Alkylreste mit 12 bis 22 Kohlenstoffatomen und besonders gerade Alkylreste mit 12 bis 22 Kohlenstoffatomen bevorzugt sind. Ganz besonders bevorzugte Alkylreste sind Lauryl, Cetyl, Cetearyl und Stearyl.

Die Polyalkylenglycolether sind durch Umsetzung der entsprechenden Fettalkohole mit Ethylenoxid (EO) erhältlich. Der Index n gibt die Anzahl an Ethylenoxidresten an. Polyalkylenglycolether mit 2 bis 21 (n = 2-21), insbesondere 2 bis 12 (n = 2-12) und ganz besonders 2 bis 5 (n = 2-5) Ethylenoxidresten sind bevorzugt.

Beispiele für erfindungsgemäß bevorzugte Polyalkylenglycolether sind Verbindungen, in denen R ein Cetylrest (C₁₆-Rest) und n 20 und insbesondere 2 ist. Diese Verbindungen haben die INCI-Bezeichnungen Ceteth-2 und Ceteth-20. Ceteth-2 hat z.B. die Formel C₁₆H₃₃-(OCH₂CH₂)₂-OH.

Weiter bevorzugt sind Verbindungen, in denen R ein Stearylrest (C₁₈-Rest) und n 2, 10, 20 oder 21 ist. Diese Verbindungen haben die INCI-Bezeichnungen Steareth-2, Steareth-10, Steareth-20 und Steareth-21. Steareth-2 hat z.B. die Formel C₁₈H₃₇-(OCH₂CH₂)₂-OH.

Ganz besonders bevorzugte nichtionische Tenside sind Steareth-20 (HLB = 15,3), Steareth-10 (HLB = 12,4), Ceteth-20 (HLB = 12,9) und insbesondere Steareth-2 (HLB = 4,9) und Ceteth-2 (HLB = 5,3).

Mischungen unterschiedlicher nichtionischer Tenside und insbesondere unterschiedlicher Polyalkylenglycolether können ebenfalls verwendet werden.

INCI steht für *International Nomenclature of Cosmetic Ingredients*. Es handelt sich um eine internationale Richtlinie für die korrekte Angabe von Inhaltsstoffen von Kosmetika. Erfindungsgemäß wurde völlig überraschend gefunden, dass die Verwendung von nichtionischen Tensiden, die üblicherweise zur Herstellung von kosmetischen und pharmazeutischen Produkten eingesetzt werden, die Eigenschaften von Schlickern für die stereolithographische Herstellung von Keramik- und Glaskeramikkörpern deutlich verbessern.

Es wurde gefunden, dass die erfindungsgemäß verwendeten nichtionischen Tenside radikalisch polymerisierbaren Monomeren und insbesondere mit Acrylaten und Methacrylaten homogen mischbar sind und die Polymerisation dieser Monomere nicht beeinträchtigen. Bei der stereolithographischen Verarbeitung der erfindungsgemäßen Schlicker im flüssigen Zustand können dadurch eine hohe Reaktivität sowie kurze Belichtungs- und Prozesszeiten gewährleistet werden. Vorteilhaft ist, dass die nichtionischen Tenside auch mit kurzkettigen polaren und stark reaktiven Monomeren gut mischbar sind.

Die Schlickerkomponenten sind oberhalb des Schmelzpunktes von Komponente (d) homogen mischbar und erstarren unterhalb des Schmelzpunktes von (d) homogen. Eine Sedimentation der Keramik- und Glaspartikel wird damit unterhalb des Schmelzpunkts der Komponente (d) wirksam verhindert. Dadurch ist die Stabilität der Mischung über lange Zeit gewährleistet.

Die erfindungsgemäßen nichtionischen Tenside sind bei Raumtemperatur fest. Dies hat zur Folge, dass sie zu einer Verfestigung der Schlicker führen, d.h. die Schlicker haben bei Raumtemperatur eine pastenförmige bis feste Konsistenz. Ein Vorteil davon ist, dass durch die homogene Erstarrung eine Sedimentation der Keramikpartikel verhindert wird und dadurch die Lagerstabilität bei Lagerung bei Raumtemperatur im festen Zustand deutlich erhöht ist. Außerdem verbessern die nichtionischen Tenside die Festigkeit der polymerisierten Objekte (Grünlinge) bei Raumtemperatur im Vergleich zu Grünlingen aus Schlickern ohne den Bestandteil (d).

Besonders vorteilhaft ist aber, dass die erfindungsgemäß verwendeten nichtionischen Tenside auch im flüssigen Zustand wirksam eine vorzeitige Sedimentation der Keramik- und oder Glaskeramikpartikel verhindern, woraus eine hohe Stabilität der Schlicker bei der Verarbeitung resultiert.

Durch die guten Dispergiereigenschaften der nichtionischen Tenside für Keramik- und Glaskeramikpartikel können die Schlicker zudem zu einem signifikant höheren Grad mit Keramik- und/oder Glaskeramikpulver gefüllt werden, was in einer höheren Gründichte resultiert.

Ein weiter Vorteil der erfindungsgemäß bevorzugten nichtionischen Tenside (d) ist, dass diese beim Entbindern schmelzen und wegen ihres relativ geringen Molekulargewichte aus der Matrix heraus fliessen. Sie behindern das Entbindern nicht, sondern schaffen vielmehr Kanäle, die den Austritt der Zersetzungsprodukte der organischen Matrix begünstigen und so das Entbindern erleichtern.

Soll bei einem polymerisierten Formteil aus einer mit keramischen Partikeln gefüllten Mischung die organische Matrix pyrolytisch entfernt werden ("Entbinderungsprozess"), so werden beim Aufheizvorgang im Ofen die zwischen den Polymerketten befindlichen Moleküle des nichtionischen Tensids (d) in flüssiger Form aus dem Formkörper ausfliessen, bevor die polymerisierten Anteile der Mischung depolymerisiert und dann pyrolysiert werden. Das Risiko von Defekten wie Rissen oder gar der Zerstörung des Formkörpers während des Entbinderungsvorgangs wird dadurch zumindest stark reduziert und meist ganz eliminiert. Zudem wird durch das Ausfliessen von Komponente (d) bereits ein Teil der organischen Masse bei vergleichsweise niedriger Temperatur entfernt, und der weitere Aufheizvorgang bis zur vollständigen Entfernung aller organischen Anteile kann zügig durchgeführt werden, während bisher nur relativ geringe Aufheizraten möglich waren.

Die erfindungsgemäßen Schlicker ergeben eine gute Grünkörperfestigkeit und hohe Formstabilität, Genauigkeit und Präzision nach dem Entbindern, Sintern und Reinigen. Beim Sintern kann ohne weiteres eine Dichte von > 98% der theoretischen Dichte erzielt werden.

Die erfindungsgemäßen Schlicker enthalten als Monomer (a) mindestens ein (Meth)acrylat und/oder (Meth)acrylamid, vorzugsweise mono- oder multifunktionelle (Meth)acrylate oder deren Mischung. Besonders bevorzugt sind Werkstoffe, die als radikalisch polymerisierbares Monomer mindestens ein multifunktionelles (Meth)acrylat oder eine Mischungen von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 6 radikalisch polymerisierbaren Gruppen verstanden.

Beispiele für besonders geeignete mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di(meth)acrylat, wie z.B. Bisphenol-A-di(meth)acrylat mit 3 (SR-348c = Methacrylat; SR-349 = Acrylat, Fa. Sartomer) oder 2 Ethoxygruppen (SR-348L = Methacrylat, Fa. Sartomer), 2,2-Bis[4-(2-(meth)acryloxypro-poxy)phenyl]propan, UD(M)A (ein Additionsprodukt aus 2-Hydroxyethyl(meth)acrylat und 2,2,4- oder 2,4,4-Trimethylhexa-methylen-1,6-diisocyanat), Di-, Tri-, Tetra-, Penta-, Hexa- oder Hepta-ethylenglycoldi(meth)acrylat, Di-, Tri-, Tetra-, Penta-, Hexa- oder Hepta-propylenglycoldi(meth)acrylat, Trimethylolpropantri(meth)acrylat, Pentaerythrittetra(meth)-acrylat, sowie Glycerindi- und -tri(meth)acrylat, 1,4-Butan-dioldi(meth)acrylat, 1,10-Decandioldi(meth)acrylat (D₃MA), 1,12-Dodecandioldi(meth)acrylat oder oligomere Polyether-, Polyester-, Epoxy-, Urethan-(meth)acrylate und Tricyclodecandimethanoldi(meth)acrylat.

Besonders bevorzugt sind mono-, di-, trifunktionelle Acrylate und Methacrylate mit einem Molekulargewicht von <1000g/mol, wie z.B. aliphatische Urethan-diacrylate, Phthalsäure-HEA-Ester (Photomer 4173), Pyromellitsäuredi-di-HEA-Ester (HEA = 2-Hydroxyethylacrylat), Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di(meth)acrylat, wie Bisphenol-A-di(meth)acrylat (SR-348c, SR-349, SR-348L), 2,2-Bis[4-(2-(meth)acryloxypropoxy)phenyl]-propan, UD(M)A, Triethylenglycoldi(meth)acrylat (TEGD(M)A), ethoxyliertes oder propopoxyliertes Trimethylolpropantri-(meth)acrylat, z.B. 3-fach propoxyliertes Trimethylolpropantriacrylat (Sartomer SR-492,) und Tripropylenglycoldiacrylat. Diese Monomere zeichnen sich durch eine hohe Reaktivität/einen hohen Doppelbindungsumsatz, gute mechanische Eigenschaften, einen geringen Polymerisationsschrumpf und eine relativ niedrige Viskosität aus.

Weiterhin geeignete Monomere sind Acrylamide wie N-Ethylacrylamid, N,N-Dimethylacrylamid, N-(2-Hydroxyethyl)acrylamid, N,N'-Diethyl-1,3-bis(acrylamido)-propan und 1,4-Bis(acryl-amido)-butan. Dabei werden die Bisacrylamide im Vergleich zu den Monoacrylamiden im organischen Bindemittel vorzugsweise im Überschuss eingesetzt.

Die Eigenschaften der Schlicker vor und nach der Lichthärtung lassen sich durch eine gezielte Kombinationen der Monomeren beeinflussen. Mischungen aus monofunktionellen und difunktionellen Monomeren zeichnen sich durch eine relativ geringe Viskosität und Reaktivität der Harzmischung aus, wobei Viskosität und Reaktivität mit dem Gehalt an monofunktionellen Monomeren abnehmen. Ein Gehalt an monofunktionellen Monomeren gewährleistet eine geringere Sprödigkeit und ein schnellere Entbinderung der durch Lichthärtung der Schlicker erhaltenen Grünlinge. Mischungen aus difunktionellen und trifunktionellen Monomeren weisen eine höhere Reaktivität auf, wobei die Reaktivität mit dem Gehalt an trifunktionellen Monomeren zunimmt. Der Gehalt an trifunktionellen Monomeren bewirkt eine höhere Sprödigkeit und langsamere Entbinderung der Grünlinge.

Reaktivität und Viskosität der Harzmischung sowie der Polymerisationsschrumpf werden weiterhin durch die Molmasse der Monomeren bestimmt, wobei mit zunehmender Molmasse der Polymerisationsschrumpf abnimmt, während die Viskosität ansteigt. Schliesslich kann über die Polarität der Monomeren die Wechselwirkung mit dem Werkstoff der stereolithographischen Wanne, wie z.B. die Quellung des Materials der Polymerisationswanne, beeinflusst werden. Als Wannenmaterialien werden häufig Siliconelastomere verwendet. Durch die Verwendung von OH-gruppenhaltigen Monomeren kann eine Quellung von Siliconelastomeren weitgehend vermieden werden.

Bevorzugte Photoinitiatoren (b) zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil. Besonders bevorzugt werden Campherchinon (CQ) und 2,2-Dimethoxy-2-phenyl-acetophenon und ganz besonders bevorzugt α-Diketone in Kombination mit Aminen als Reduktionsmittel eingesetzt, wie z.B. 4-(Dimethylamino)-benzoesäureester (EDMAB), N,N-Dimethylaminoethylmethacrylat, N,N-Dimethyl-sym.-xylidin oder Triethanolamin.

Besonders bevorzugte Photoinitiatoren sind Norrish-Typ-I-Photoinitiatoren, vor allem Monoacyl- oder Bisacylphosphinoxide, und insbesondere Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethyl-germanium (MBDEGe). Dabei lassen sich vorteilhaft auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Ganz besonders bevorzugt sind Campherchinon (CAS Nr. 10373-78-1) in Kombination mit Ethyl-4-(dimethylamino)benzoat (EMBO, CAS Nr. 10287-53-3), sowie Norrish Typ I Photoinitiatoren, insbesondere 2,4,6-Trimethylbenzoyldipenylphosphinoxid (TPO, CAS Nr. 75980-60-8), Ethyl(2,4,6-trimethylbenzoyl)phenylphos-phinat (TPO-L, CAS Nr. 84434-11-7), Phenylbis(2,4,6-trimethylbenzoyl)phosphinoxid (Irgacure 819, CAS 162881-26-7), Bis(2,6-difluoro-3-(1-hydropyrrol-1-yl)phenyl)titanocen (Irgacure 784, CAS Nr. 125051-32-3), 2-Benzyl-2-(dimethylamino)-4'-morpholinobutyrophenon (Irgacure 369, CAS Nr. 119313-12-1), 1-Butanon-2-(dimethylamino)-2-(4-methylphenyl)methyl-1-4-(4-morpholinyl)phenyl (Irgacure 379, CAS-Nr. 119344-86-4) und ganz besonders Bis(4-methoxybenzoyl)diethylgermanium (MBDEGe; K69).

Als Komponente (c) enthalten die erfindungsgemäßen Schlicker Keramik- und/oder Glaskeramikpartikel.

Unter Keramiken werden anorganische Werkstoffe verstanden, die eine kristalline Struktur aufweisen und meist aus entsprechenden Pulvern hergestellt werden. Vorzugsweise erfolgt die Herstellung der Keramik durch Sintern (Sinterkeramik). Oxidkeramiken werden vorzugsweise durch Sintern von Metalloxidpulvern wie z.B. ZrO₂ oder Al₂O₃ erhalten. Darüber hinaus können Oxidkeramiken auch eine oder mehrere Glasphasen enthalten.

Bevorzugt sind Keramikpartikel auf der Basis von ZrO₂ oder Al₂O₃, vorzugsweise reinem ZrO₂ oder reinem Al₂O₃, Partikel auf der Basis von mit HfO₂, CaO, Y₂O₃, CeO₂ und/oder MgO stabilisiertem ZrO₂, Partikel auf der Basis von anderen Metalloxiden sowie keramischen Kompositmaterialien, die aus mehreren Oxiden hergestellt werden und somit aus unterschiedlichen kristalline Oxidphasen aufgebaut sind, vorzugsweise ZrO₂-Al₂O₃, insbesondere reinem ZrO₂-Al₂O₃ oder mit HfO₂, CaO, Y₂O₃, CeO₂ und/oder MgO stabilisiertem ZrO₂-Al₂O₃.

Der Begriff "rein" ist im Sinne von "chemisch rein" zu verstehen, d.h. eine ZrO₂- bzw. Al₂O₃-Keramik ist nur aus ZrO₂ bzw. Al₂O₃ aufgebaut. Stabilisierte Keramiken enthalten neben dem Basisoxid wie ZrO₂ oder Al₂O₃ ein Stabilisisierungsmittel, das vorzugsweise aus HfO₂, CaO, Y₂O₃, CeO₂, MgO und Mischungen davon ausgewählt ist. Das Stablilisierungsmittel wird vorzugsweise in einer Menge von 3 bis 5 Gew.-% eingesetzt, bezogen auf die Masse der stabilisierten Keramik. Hochfeste ZrO₂-Keramiken enthalten zur Stabilisierung der tetragonalen Kristallstruktur vorzugsweise 3 bis 5 Gew.-% Y₂O₃ (Yttriumoxid) . Diese ZrO₂-Keramik wird als Y-TZP (Yttrium Stabilized Tetragonal Zirconium Dioxide Polycrystals) bezeichnet. Keramikpartikel, die nur Basisoxid und Stabilisierungsmittel enthalten, sind besonders bevorzugt.

Erfindungsgemäß sind Schlicker bevorzugt, die als Komponente (c) Glaskeramikpartikel enthalten. Bei Glaskeramiken handelt es sich um Werkstoffe, die meist aus amorphen Gläsern, insbesondere Silikatgläsern, durch gesteuerte Kristallisation hergestellt werden und bei denen eine Glasphase und eine oder mehrere Kristallphasen im Festkörper nebeneinander vorliegen. Bei Glaskeramiken kann sowohl von Glaspulvern als auch von Glaskeramikpulvern ausgegangen werden. Besonders bevorzugt sind Glaskeramikpartikel, die Leucit- und ganz besonders bevorzugt Lithiumdisilikatkristalle enthalten. Diese lassen sich vorteilhaft aus Lithiumdisilikat-Glaspulver, ohne Anteile an Kristalliten, durch thermische Behandlung (Kristallisations- und Sinterbrand) herstellen.

Die als Komponente (c) eingesetzten Keramik- oder Glaskeramikpartikel sind vorzugsweise eingefärbt. Hierzu werden die Keramik- und/oder Glaskeramikpartikel vorzugsweise mit einem oder mehreren Pigmenten gemischt. Besonders bevorzugt sind anorganische Pigmente, wie beispielsweise die Oxide von Pr, Tb, Ce, Co, Ni, Cu, Bi, La, Nd, Sm, Eu, Gd und insbesondere von Fe, Mn, Cr, Er. Zur Einfärbung der Keramik- oder Glaskeramikpulver können vorteilhaft auch farbige Spinell-Verbindungen des Typs AB₂O₄ verwendet werden, wobei A bevorzugt ein Alkali- oder Erdalkalimetallion und B ein Übergangsmetallion höherer Oxidationsstufe als A ist. Die Spinelle eignen sich besonders zur Einfärbung von Glaskeramik und insbesondere von Lithiumdisilikatglaskeramik. Die farbgebenden Komponenten werden einer Menge zugesetzt, die erforderlich ist, um die gewünschte Färbung zu erzielen. Vorzugsweise werden Art und Menge der farbgebenden Komponente(n) so gewählt, dass nach dem Entbindern und Sintern zahnfarbene Keramikformteile erhalten werden. Der Farbeindruck wird erst während des Sinterns endgültig ausgebildet. Üblicherweise werden die farbgebenden Komponenten in einer Menge von jeweils 0,01 bis 1 Gew.-%, bevorzugt 0,05 bis 0,5 Gew.-% bezogen auf die Gesamtmasse der Komponente (c) eingesetzt.

Zur Erzielung des gewünschten Farbeindrucks ist es auch möglich, Mischungen von unterschiedlich eingefärbten Keramik- oder Glaskeramikpulvern einzusetzen.

Die Partikelgröße der Komponente (c) liegt vorzugsweise im Bereich von 10 nm bis 100 pm, vorzugsweise 100 nm bis 10 µm. Sie ist abhängig von der eingesetzten Keramik. Bei Al₂O₃ liegt die Größe der als Komponente B verwendeten Partikel vorzugsweise im Bereich von 50 bis 500 nm, besonders bevorzugt zwischen 75 und 200 nm; bei Glaskeramik im Bereich von 500 nm bis 50 pm, ganz bevorzugt zwischen 1 und 10 pm; bei TZP-3Y Zirkondioxid im Bereich von 50 bis 500 nm, ganz bevorzugt zwischen 50 und 350 nm. Die Partikelgröße wird dabei vorzugsweise so gewählt, dass sedimentationsstabile Schlicker erhalten werden. Bei den Partikelgrößen handelt es sich um die absoluten Ober- und Untergrenzen.

Weiterhin lassen sich auch Keramik- oder Glaskeramikpartikel mit einer Partikelgröße im Bereich von 10-200 nm als Nano- der Organosole, d.h. als Dispersion der Nanopartikel in einem Lösungsmittel, einem geeigneten Monomer der Komponente (a) oder einer Mischungen daraus einsetzen.

Gemäß einer besonders bevorzugen Ausführungsform der Erfindung werden die Partikel der Komponente (c) mit geeigneten Stoffen oberflächenmodifiziert. Zur Oberflächenmodifizierung werden vorzugsweise solche Verbindungen eingesetzt, die chemisch, d.h. durch ionische oder kovalente Bindungen an die Oberfläche der Keramik- oder Glaskeramikpartikel gebunden werden. Bevorzugt sind Verbindungen, die entweder Säuregruppen, vorzugsweise Carbonsäure-, Phosphonsäure-, Hydrogenphosphatgruppen oder saure Phosphorsäureestergruppen, oder Silylgruppen, vorzugsweise Alkoxysilylgruppen enthalten. Die Partikeloberfläche kann teilweise oder vorzugsweise vollständig mit dem Modifizierungsmittel bedeckt sein. Bei den erfindungsgemäß verwendeten Modifizierungsmitteln handelt es sich um monomere Verbindungen.

Erfindungsgemäß sind solche Verbindungen besonders geeignet, die im Gegensatz zu den sogenannten Haftvermittlern oder Kopplungsreagenzien nur mit der Partikeloberfläche reagierende Gruppen enthalten, aber keine radikalisch polymerisierbaren Gruppen, die mit der Harzmatrix (a) eine kovalente Bindung eingehen. Solche Verbindungen werden hierin als nicht polymerisierbare Oberflächenmodifizierungsmittel bezeichnet. Diese Verbindungen haben den Vorteil, dass ein stabiler Verbund zwischen der Keramikpartikeloberfläche und der Polymermatrix im ausgehärteten Grünling nicht zustande kommt, was die vollständige Entfernung der Polymeranteile im Entbinderungsprozess vereinfacht.

Als nicht polymerisierbare Oberflächenmodifizierungsmittel eigen sich insbesondere lineare oder verzweigte Carbonsäuren, wie z.B. Ameisensäure, Essigsäure, Propionsäure, Octansäure, Isobuttersäure, Isovaleriansäure, Pivalinsäure oder Phosphonsäuren, z.B. wie Methyl-, Ethyl-, Propyl-, Butyl- Hexyl, Octyl- oder Phenylphosphonsäure. Als nicht polymerisierbare Oberflächenmodifikatoren geeignete Silane sind beispielsweise Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan. Ganz besonders bevorzugt sind saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat.

Als Oberflächenmodifizierungsmittel eignen sich für wässrige Schlicker weiterhin Polymere, insbesondere Polyelektrolyte, z.B. Polycarbonsäuren oder Polycarbonsäuresalze, oder nichtionische Polymere, wie z.B. Polyethylenglycol oder Carboxymethylcellulose. Polyelektrolyte, die wie z.B. Ammoniumpolycarboxylat ionische Gruppen tragen, können relativ leicht an die Oberfläche von Festkörpern adsorbieren und den Partikeln dabei eine elektrische Ladung verleihen. Im Falle von organischen, nicht wässrigen Schlickern eignen sich Polymere, die im Polyreaktionsharz löslich sind. Modifizierungsmittel mit einer Molmasse im Bereich von 50 bis 5.000 g/mol sind bevorzugt.

Oberflächenmodifizierungsmittel werden ggf. in einer Menge von vorzugsweise 0,1 bis 5 Gew.-%, besonders bevorzugt 0,2 bis 2 Gew.-% und ganz besonders bevorzugt 0,5 bis 1,5 Gew.-% bezogen auf die Masse des Schlickers eingesetzt.

Neben den oben genannten Komponenten enthalten die erfindungsgemäßen Schlicker vorzugsweise zusätzlich mindestens ein Additiv, das aus Farbstoffen, Lösungsmitteln, Inhibitoren, Entbinderungsbeschleunigern, Entschäumungsmitteln und/oder Hautverhinderungsmitteln ausgewählt ist.

Als Farbstoff sind organische Farbstoffe bevorzugt, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in dem Schlicker löslich sind, insbesondere Azofarbstoffe. Ein ganz besonders bevorzugter Farbstoff ist (4-(4-Nitrophenylazo)anilin (Disperse Orange 3, CAS Nr. 730-40-5). Als farbgebende Komponente (e) sind solche Stoffe bevorzugt, die im gleichen Wellenlängenbereich absorbieren wie der Polymerisationsinitiator. Besonders bevorzugt sind Farbstoffe, die ein Absorptionsmaximum aufweisen, das der Wellenlänge des zur Härtung eingesetzten Lichts entspricht. Sehr vorteilhaft sind Farbstoffe mit einem Absorptionsmaximum im Bereich von 350 bis 550 nm, vorzugsweise 380 bis 480 nm.

Anders als bei den zur Einfärbung der Keramik- bzw. Glaskeramikpulver verwendeten farbgebenden Komponenten werden unter Farbstoffen hier solche Stoffe verstanden, die beim Entbindern und Sintern vollständig verbrennen. Diese Farbstoffe dienen allein dazu, den Schlicker einzufärben und so dessen Verarbeitung zu erleichtern. Sie dienen nicht zur Einfärbung der Keramik.

Als Lösungsmittel sind organische Lösungsmittel bevorzugt, insbesondere solche Lösungsmittel, die eine Siedetemperatur von mindestens ca. 120 °C, vorzugsweise von 150 bis 250 °C aufweisen, so dass es bei der stereolithographischen Verarbeitung des Schlickers nicht zu einer vorzeitigen Verdunstung kommt. Dabei sind Mischungen von solchen Lösungsmitteln besonders geeignet, die in einem Temperaturbereich zwischen 150 und 250 °C schrittweise verdampfbar sind. Ganz besonders geeignet sind n-Octanol, Triethylenglycol-divinylether, 2-Amino-2-methyl-1-propanol, 2-Methyl-2,4-pentandiol, Tripropylenglycol, Tetraethylenglycol, Triethylenglycol, Triethylcitrat, Acet-Essigsäure-Ethylester, Cyclohexanol, Cyclohexanon, Diethylenglycolmonomethylether, Oxalsäuredibutylester, 2,5-Dimethoxytetrahydrofuran, Polythylenlycol 300, 1- oder 2-Nonanol, Diethylenglycoldiethylether, 2,5-Dimethoxytetrahydrofuran, Oxalsäuredibutylester, Cyclohexanol, Cyclohexanon, Acetessigsäureethylester und Mischungen davon.

Bevorzugte Lösungsmittel sind außerdem Polyethylenglykole (PEG), Polypropylenglykole (PPG), PEG-PPG-Co-Polymere, Glycerin und Glycerinderivate, d.h. insbesondere ethoxyliertes und/oder propoxyliertes Glycerin, sowie Phthalate. Besonders bevorzugt sind Lösungsmittel mit einem Mw kleiner als 5.000 g/mol, bevorzugt kleiner als 1.000 g/mol. Als Lösungsmittel eignen sich Verbindungen, die bei Raumtemperatur flüssig sind und die mit den übrigen Komponenten des Schlickers unter Lager- und Anwendungsbedingungen nicht reagieren. Besonders bevorzugte Lösungsmittel sind PEG 200-600g/mol, PPG 200-800g/mol, Co-PEG-PPG 200-800g/mol und insbesondere Polypropylenglycol ∼400g/mol. Es können vorteilhaft auch Mischungen dieser Lösungsmittel eingesetzt werden.

Das oder die Lösungsmittel werden vorzugsweise in einer Gesamtmenge von 0 bis 50 Gew.-%, besonders bevorzugt von 5 bis 20 Gew.-% eingesetzt, bezogen auf die Masse des Schlickers.

Es wurde gefunden, dass das Verdampfen der obigen Lösungsmittel die Bildung von Mikroporen im Grünling zusätzlich fördert. Diese Poren schließen sich ebenso wie die durch das Ausfließen der nichtionischen Tenside gebildeten Kanäle beim Sintern. Sie erleichten das Entweichen der Gase im beim Entbindern und verhindern so das Entstehen von Spannungen und Rissen. Außerdem wird die Gefahr einer Trennung der stereolithographisch erzeugten Schichten reduziert und eine vollständige Entfernung aller organischen Komponenten begünstigt.

Alternativ kann die Porosität des Grünlings auch dadurch vergrößert werden, dass vor der Wärmebehandlung herauswaschbare Anteile extraktiv entfernt werden. Geeignete extrahierbare Komponenten sind wasserlösliche Polymere, wie z.B. Polyvinylalkohol, Polyvinylpyrrolidon und Polyethylenglycole. Weiterhin können benzinlösliche Stoffe wie z.B. langkettige Fettsäureester eingesetzt werden. Die bevorzugte Menge an extrahierbaren Komponenten liegt zwischen 0 und 30 Gew.-%, besonders bevorzugt zwischen 1 und 20 Gew.-%, bezogen auf die Masse des Schlickers.

Die erfindungsgemäßen Schlicker können vorteilhaft als weiteres Additiv einen Inhibitor zur Verhinderung einer spontanen Polyreaktion als Stabilisator enthalten. Die Inhibitoren bzw. Stabilisatoren verbessern die Lagerstabilität der Schlicker und verhindern darüber hinaus eine unkontrollierte Polyreaktion in der stereolithographischen Wanne. Die Inhibitoren werden vorzugsweise in einer solchen Menge zugesetzt, dass die Schlicker über einen Zeitraum von ca. 2-3 Jahre lagerstabil sind. Besonders bevorzugt werden die Inhibitoren in einer Menge von 0,001 bis 1,0 Gew.-%, ganz besonders bevorzugt 0,001 bis 0,50 Gew.-% eingesetzt, jeweils bezogen auf die Gesamtmasse des Schlickers.

Bevorzugt sind sog. aerobe Inhibitoren Phenole, wie Hydrochinonmonomethylether (MEHQ) oder 2,6-Di-tert.-butyl-4-methylphenol (BHT), eingesetzt, die nur in Gegenwart von Sauerstoff effektiv wirksam sind und vorzugsweise in einem Konzentrationsbereich von 100-2000 ppmw verwendet werden. Geeignete anaerobe Inhibitoren sind Phenothiazin, 2,2,6,6-Tetramethyl-piperindin-1-oxyl-Radikal (TEMPO), Iod und Kupfer-(I)-iodid. Diese wirken schon in geringen Konzentrationen von vorzugsweise 10-50 ppm auch bei Abwesenheit von Sauerstoff. Eine Polymerisation findet erst dann statt, wenn diese Additive verbraucht sind. Dabei ist es von Vorteil, eine Mischung von aeroben und anaeroben Inhibitoren einzusetzen.

Aerobe Inhibitoren werden vorzugsweise in einer Menge von 0,001 bis 0,50 Gew.-% und anaerobe Inhibitoren in einer Menge von 0,001 bis 0,02 Gew.-% eingesetzt, jeweils bezogen auf die Gesamtmasse des Schlickers. Bevorzugte Mischungen enthalten 0,005-0,10 Gew.-% an aeroben Inhibitoren und 0,001- bis 0,01 Gew.-% an anaeroben Inhibitoren, ebenfalls bezogen auf die Gesamtmasse des Schlickers.

Gemäß einer weiteren Ausführungsform der Erfindung enthalten die Schlicker als zusätzliches Additiv einen sogenannten Entbinderungsbeschleuniger. Dieser wird vorzugsweise in einer Menge von 0 bis 20 Gew.-%, besonders bevorzugt 0,01 bis 10 Gew.-% eingesetzt, jeweils bezogen auf die Gesamtmasse des Schlickers. Unter Entbindungsbeschleunigern werden Stoffe verstanden, welche das Entfernen des Bindemittels während des Entbinderungsprozesses erleichtern.

Die Entbinderung des Grünlings kann z.B. durch polyreaktionswirksame Stoffe im Polyreaktionsharz gefördert oder zielgerichtet beeinflusst werden. Dabei handelt es sich einerseits um Additive, die die Netzwerkbildung beeinflussen, wie vor allem kettenübertragungsaktive Stoffe, sog. Kettenregler, die zu einer Verringerung der Polymernetzwerkdichte und damit zu einer besseren thermischen Abbaubarkeit führen. Bekannte Kettenregler z.B. für die radikalische Polymerisation sind vor allem Mercaptane, wie z.B. Laurylmercaptan, und Disulfide. Disulfide, vor allem Dithiourethandisulfide, wie z.B. Tetramethylthiuramdisulfid oder Isopropylxanthogensäuredisulfid wirken bei der radikalischen Photopolymerisation als sog. Photoiniferter. Es handelt sich dabei um Verbindungen, die sowohl als Photoinitiator (Photoini-) wirken, als auch an Übertragungsreaktionen (engl.: transfer, -fer-) und Abbruchsreaktionen (engl.: termination, -ter) teilnehmen (vgl. vgl. T. Ostsu, M. Yoshida, Makromol. Chem., Rapid. Commun. 3 (1982) 127-132: Role of Initiator-Transfer Agent-Terminator (Iniferter) in Radical Polymerizations: Polymer Design by Organic Disulfides as Iniferters). Die Zugabe von kettenübertragungsaktiven Stoffen, d.h. von Kettenreglern oder Photoinifertern, bewirkt eine Verringerung der Netzwerkdichte des Polyreaktionsnetzwerkes, bei nahezu unveränderter Reaktivität der Polyreaktionsharzmischung. Kettenregler und Photoiniferter werden vorzugsweise in einer Menge von jeweils 0,005 bis 25 Gew.-% und besonders bevorzugt 0,01 bis 10 Gew.-% bezogen auf die Komponente (a) eingesetzt.

Als Entbinderungsbeschleuniger lassen sich auch Comonomere einsetzen, die zu einer Verringerung der thermischen Stabilität von Polymernetzwerken führen. Hierzu eignen sich Comonomere, die thermisch labile Gruppen enthalten, wie z.B. Peroxid-, Azo- oder Urethangruppen, welche während des stereolithographischen Prozesses in das Polymernetzwerk eingebaut werden und dann im thermischen Entbinderungsprozess den Abbau des Polymernetzwerkes beschleunigen. Ein bevorzugtes Beispiel für ein polymerisationsfähiges Peroxid ist 4,4'-Divinylbenzoylperoxid, das durch Umsetzung von 4-Vinylbenzoylchlorid mit Natriumperoxid zugänglich ist. Ein bevorzugtes Beispiel für eine polymerisationsfähige Azo-Verbindung ist der Ester aus 2-Hydroxyethylmethacrylat und der 4,4'-Azobis-(4-cyano-valeriansäure). Bevorzugte thermisch labile Urethane sind aus Diisocyanaten zugänglich, beispielsweise durch Umsetzung von 2,2,4-Trimethylhexamethylendiisocyanat (TMDI) oder Toluylendiisocyanat (TDI) mit Hydroxypropylacrylat (HPA) oder 2-Hydroxyethylacrylat (HEA). Ein weiteres Beispiel für einen thermisch labilen Monomerbaustein stellt α, α, α', α' -Tetramethyl-1,4-ben-zen-dimethylacrylat dar, dessen Einbau in ein Michael-Addition-Netzwerke beispielsweise von Diacrylaten und Diacetoacetaten in Gegenwart katalytischer Säuremengen zu einem beschleunigten Abbau des Polymernetzwerks führt.

Außerdem sind als Entbinderungsbeschleuniger Comonomere geeignet, deren Polyreaktionsprodukte thermisch leicht abbaubar sind. Für radikalische Polymerisationsharze sind Comonomere bevorzugt, die wie α-Methylstyrol eine niedrige ceiling-Temperatur T_{c} aufweisen. Die ceiling-Temperatur ist die Grenztemperatur, bei der die Polymerisation mit der Depolymerisation im Gleichgewicht steht, und lässt sich aus dem Quotienten der Polymerisationsenthalpie und der Polymerisationsentropie berechnen (vgl. H.-G. Elias, Makromoleküle, Bd 1, 6. Aufl., Wiley-VCH, Weinheim etc. 1999, 193ff.). Beispielsweise beträgt T_{c} für α-Methylstyrol 61°C. Die ceiling-Temperatur T_{c} von Polytetrahydrofuran (PTHF) liegt bei 80°C. Dementsprechend kann z.B. durch Verwendung von Telechelen, insbesondere PTHF-Di(meth)acrylat-Telechelen als Comonomer die Abbaubarkeit von Poly(meth)acrylatnetzwerken beschleunigt werden. Erfindungsgemäß sind Comonomere mit einer ceiling-Temperatur von -10 bis 150°C, vorzugsweise 10 bis 150°C und besonders bevorzugt 20 bis 130°C besonders geeignet. Die Comonomere werden vorzugsweise in einer Menge von 0,1 bis 30 Gew.-% und besonders bevorzugt 0,5 bis 20 Gew.-% bezogen auf die Komponente (a) eingesetzt.

Weiterhin können die erfindungsgemäßen Schlicker Additive enthalten, die beim Entbinderungsprozess den oxidativen Abbau der Polymermatrix fördern, wie z.B. bei Raumtemperatur stabile Peroxide, oder auch katalytisch wirksame Komponenten, die eine katalytische Entbinderung ermöglichen. Neben Peroxiden eignen sich auch andere Substanzen, die oxidierend wirken, wie z.B. Salpetersäure, oder die Oxidationsmittel abspalten oder bilden.

Zusätzlich können die erfindungsgemäß Schlicker Entschäumungsmittel und/oder Hautverhinderungsmittel enthalten, die die Schaumbildung bei der Herstellung der Schlicker bzw. die Bildung einer Haut während der Verarbeitung der Schlicker verhindern. Entschäumungs- und/oder Hautverhinderungsmittel werden vorzugsweise in eine Menge von jeweils 0 bis 5 Gew.-%, besonders bevorzugt 0,1 bis 2 Gew.-% in der organischen Matrix eingesetzt, bezogen auf die Masse der Komponente (A).

Die rheologischen Eigenschaften der erfindungsgemäßen Schlicker werden vorzugsweise so eingestellt, dass ihre Viskosität im Bereich von 200-20.000 mPas, besonders bevorzugt 500-5.000 mPas liegt. Die Viskosität wird bei der gewünschten Verarbeitungstemperatur des Schlickers mit einem Platte-Platte-Viskosimeter bestimmt. Die Verarbeitungstemperatur liegt vorzugsweise im Bereich von 30-100°C, besonders bevorzugt 40-70°C.

Die erfindungsgemäßen Schlicker weisen vorzugsweise folgende Zusammensetzung auf:
- 5 bis 65 Gew.-%, vorzugsweise 9 bis 57 Gew.-%, besonders bevorzugt 10 bis 40 Gew.-% Monomer (a);
- 0,001 bis 1,0 Gew.-%, vorzugsweise 0,01 bis 1,0 Gew.-%, besonders bevorzugt 0,1 bis 1,0 Gew.-% Photoinitiator (b);
- 33 bis 90 Gew.-%, vorzugsweise 40 bis 88 Gew.-%, besonders bevorzugt 56 bis 86 Gew.-% Keramik- und/oder Glaskeramikpartikel (c);
- 1 bis 30 Gew.-%, vorzugsweise 2 bis 15 Gew.-%, besonders bevorzugt 3 bis 10 Gew.-% nichtionisches Tensid (d).

Weiterhin enthalten die Schicker vorzugsweise:
- 0 bis 0,2 Gew.-%, vorzugsweise 0 bis 0,05 Gew.-%, besonders bevorzugt 0 bis 0,02 Gew.-% Farbstoff(e) zur Einfärbung der Schlicker; und/oder
- 0,1 bis 5 Gew.-%, vorzugsweise 0,2 bis 2 Gew.-%, besonders bevorzugt 0,5 bis 1,5 Gew.-% Oberflächenmodifizierungsmittel; und/oder
- 0 bis 50 Gew.-%, vorzugsweise 5 bis 20 Gew.-%, besonders bevorzugt 5 bis 10 Gew.-% Lösungsmittel.

Wenn nicht anders angegeben beziehen sich alle Angaben auf das Gesamtgewicht des Schlickers. Die zur Einfärbung der Keramik- oder Glaskeramikpartikel verwendeten farbgebenden Komponenten sind in den für die Komponente (c) genannten Mengen enthalten.

Die erfindungsgemäßen Schlicker eignen sich besonders zur Herstellung von Keramik- oder Glaskeramikformteilen, insbesondere zur Herstellung von Dentalrestauration, wie z.B. Inlays, Onlays, Veneers, Kronen, Brücken oder Gerüsten.

Gegenstand der Erfindung ist weiterhin ein Verfahren zur Herstellung von Keramik- oder Glaskeramikformteilen bei dem man
(A) einen Grünling herstellt, indem man einen erfindungsgemäßen Schlicker durch lokales Einbringen von Strahlungsenergie unter Ausbildung der geometrischen Form des Grünlings aushärtet,
(B) man dann den Grünling einer Wärmebehandlung zur Entfernung des Bindemittels (Entbinderung) unterwirft, um einen Weissling zu erhalten, und
(C) man den Weissling anschließend sintert.

Die Herstellung des Grünlings in Schritt (A) erfolgt durch Stereolithographie. Es wird durch schichtweise Strahlungshärtung eines bei der Verarbeitungstemperatur fließfähigen keramischen Schlickers ein keramischer Grünling hergestellt, der in Schritt (B) entbindert wird. Hierbei wird das verwendete Bindemittel durch Erhitzen des Grünlings auf eine Temperatur von vorzugsweise 90°C bis 600°C entfernt, und es wird der sogenannte Weißling erhalten. Der Weißling wird in Schritt (C) zu einem dichten keramischen Formkörper gesintert. Die Sinterung des Weißlings erfolgt im Sinterofen, vorzugsweise bei einer Temperatur für Glaskeramik von 650 bis 1100°C, bevorzugt 700 bis 900°C, für Zirkondioxid von 1100 bis 1600°C und für Aluminiumoxid von 1400 bis 1800°C, bevorzugt 1600 bis 1700°C. Die nach dem erfindungsgemäßen Verfahren hergestellten Keramikformkörper zeichnen sich durch eine hohe Festigkeit und große Detailgenauigkeit aus. Die Biegefestigkeit nach ISO 6872 liegt bei Formkörpern aus Glaskeramik vorzugsweise über 100 MPa, insbesondere im Bereich von 150 bis 500 MPa. Formkörper aus Al₂O₃ haben eine Biegefestigkeit von vorzugsweise über 300 MPa, insbesondere von 500 bis 700 MPa, Formkörper aus ZrO₂ von mehr als 500 MPa insbesondere von 800 bis 1100 MPa.

Die Erfindung wird im Folgenden anhand von Zeichnungen und Ausführungsbeispielen näher erläutert.

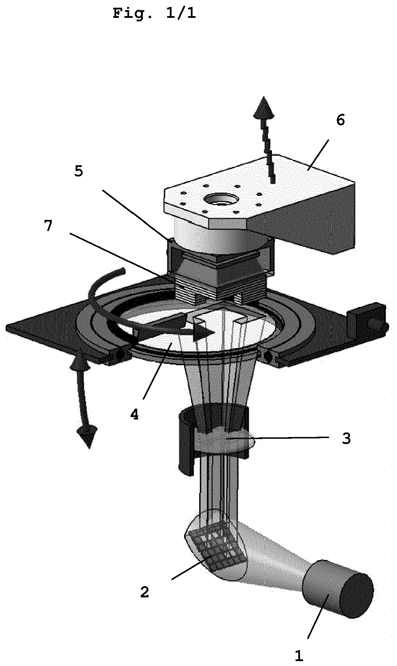

**Fig. 1** zeigt eine schematische Darstellung einer Vorrichtung zur Durchführung des erfindungsgemäßen Verfahrens. Wie Fig. 1 zu entnehmen ist, umfasst die Vorrichtung einen Behälter **4** für den erfindungsgemäßen Schlicker. Der Behälter **4** wird auch als Polymerisationswanne oder Wanne bezeichnet. In der dargestellten Ausführungsform weist die Wanne **4** ein transparentes Fenster auf, durch das der Schlicker von unten selektiv belichtet und ausgehärtet wird. Unterhalb der Wanne **4** ist ein computergesteuerter, DMD-Chip (Digital Mirror Devices) **2** angeordnet, der mit einer Lichtquelle **1** bestrahlt wird. Das Bild des Spiegels **2** wird durch eine optische Vorrichtung **3** auf das transparente Fenster im Boden des Behälters **4** projiziert. Über der Wanne **4** ist ein in Z-Richtung verfahrbarer Substratträger **6** angeordnet, der den schichtweise aufgebauten Körper **7** trägt. Der Substratträger **6** weist eine Trägerplatte **5** auf. Die Trägerplatte **5** wird soweit in den Schlicker eingetaucht, dass der Abstand zwischen der Trägerplatte **5** bzw. dem daran befestigen Körper **7** und der inneren Oberfläche der Wanne **4** der Schichtdicke der zu erzeugenden Schicht entspricht. Anschließend wird die Schlickerschicht zwischen Trägerplatte und innerer Wannenoberfläche durch das transparente Fenster hindurch mit Hilfe des DMD-Chips **2** selektiv belichtet und gehärtet. Es entstehen gehärtete Bereiche, die an der Trägerplatte **5** anhaften. Anschließend wird der Träger **6** in Z-Richtung nach oben bewegt, so dass zwischen der anhaftenden Schicht und der inneren Wannenoberfläche wieder eine Schlickerschicht mit der gewünschten Dicke entsteht. Durch erneute Belichtung wird auch diese Schicht selektiv gehärtet und durch Wiederholung des Vorgangs der gewünschte Formkörper, vorzugsweise eine dentale Restauration, schichtweise aufgebaut.

### Ausführungsbeispiele

### Beispiel 1

### Lithiumdisilikat-Schlicker

Die in Tabelle 1 aufgeführten Komponenten wurden bis auf die Glaskeramikpartikel eingewogen und bei 50°C gerührt bis alle Komponenten gelöst waren. Dann wurde die Pulverkomponente (c) in 2-3 Chargen zugegeben. Nach jeder Pulverzugabe wurde der Schlicker in einem Hauschild Speedmixer DAC 400fvz bei 2750rpm für 2min homogenisiert. Das Gesamtgewicht der Schlicker betrug jeweils ca. 50-100g.

| **Komponente** | **Stoff** | **Schlicker 1** | **Schlicker 2** | **Schlicker 3*)** |
|---|---|---|---|---|
| Monomer (a) | 3-fach propoxyliertes Trimethylolpropantriacrylate¹⁾ | 8.65% | 8% | 8.65% |
| Monomer (a) | UDMA (RM3) | 6.8% | 6% | 6.8% |
| Initiator (b) | Ivocerin²⁾ | 0.05% | 0.05% | 0.05% |
| nichtion. Tensid (d) | Steareth-2³ | 9.15% | 3.9% | - |
| Farbstoff | 4-(4-Nitrophenylazo)anilin 3⁴⁾ | 0.005% | 0.005% | 0.005% |
| Dispergiermittel | Phosphorsäureester⁵⁾ | 1% | 1% | 1% |
| Lösungsmittel | Polypropylene Glycol (400 g/mol) | - | 6.7% | 9.15% |
| Glaskeramikpartikel (c) | Lithiumdisilikat⁶⁾ | 74.345% | 74.345% | 74.345% |

| | | | | |
|---|---|---|---|---|
| *) Vergleichsbeispiel ¹⁾ SR492 (Firma Sartomer) ²⁾ Bis(4-meth¬oxy¬benzoyl)di¬ethyl¬ger¬manium ³⁾ Brij S2 (Firma Croda Europe Ltd, England) ⁴⁾ Disperse Orange 3 (Firma Sigma-Aldrich; CAS Nr. 730-40-5) ⁵⁾ Solplus D540 (CAS 1000871-74-8; Firma Lubrizol) ⁶⁾ Lithiumdisilikatglaskeramik (e.max Transpa; Firma Ivoclar Vivadent, Liechtenstein), gemahlen, D50 = µm | | | | |

Die Schlicker 1 bis 3 sind Lithiumdislikat Schlicker. Schlicker 1 enthält kein Lösungsmittel. Bei Schlicker 2 ist zusätzlich Lösungsmittel (PPG400) vorhanden, und der Gehalt an nichtionischem Tensid (d) ist reduziert. Damit wird der Schlicker unempfindlicher gegenüber Temperaturschwankung während der Verarbeitung, ist dafür aber bei Raumtemperatur weniger fest und weicher. Schlicker 3 (Vergleichsbeispiel) enthält kein nichtionisches Tensid.

Das nichtionische Tensid bewirkt eine Erstarrung des Schlickers bei Raumtemperatur, wodurch die Lagerstabilität deutlich erhöht wird. Nach 3 Wochen waren bei Schlicker 1 und 2 visuell keine Sedimentation und keine Phasenseparation sichtbar. Schlicker 3 sedimentierte innerhalb von 3 Wochen deutlich und es bildete sich an der Oberfläche ein gut sichtbarer, transparenter Flüssigkeitsfilm aus. Der sedimentierte Schlicker war durch manuelles Schütteln oder Rühren nicht wieder homogenisierbar, und ließ sich auch durch intensives maschinelles Aufrühren (Speedmixer) nur bedingt wieder homogenisieren. Diese Möglichkeiten sind allerdings auf dem SL-Gerät nicht möglich.

### Beispiel 2

### Wachshaltiger Schlicker (Vergleichsbeispiel)

### Zusammensetzung des Schlickers

| **Komponente** | **Menge** | **Anteil** |
|---|---|---|
| ZrO₂-Partikel | 150 g | (35,7 Vol.-%; 78,2 Gew-%) |
| Paraffinwachs | 20,310 g | (32,7 Vol.%; 10,6 Gew.-%) |
| PPG700DA | 11,858 g | (16,8 Vol.-%; 6, 2 Gew.-%) |
| 5-DAMS | 5,442 g | (7,7 Vol.-%; 2,8 Gew.-%) |
| Octadecen | 2,020 g | (3,7 Vol.-%; 1,1 Gew.-%) |
| Hypermer LP-1 | 2,000 | (3,2 Vol.-%; 1,0 Gew.-%; ) |
| TEMPO | 0,001 g | (0,03 Vol.-%; 5 Gew.-ppm) |
| Photoinitiator | 0,300 g | (0,017 Vol.-%; 0,15 Gew.-%) |

Erläuterung:
- ZrO₂: TZ-3YS-E (commercial grade) von Tosoh Corporation, Tokyo, JP (mit Y₂O₃ stabilisiertes ZrO₂, Primärteilchengröße 300-350 nm)
- Paraffinwachs: Schmelzpunkt 54-56°C, Viskosität (bei 80°C und einer Scherrate von 1000 s⁻¹) 3-4 mPa·s (Siliplast; Zschimmer & Schwarz, Lahnstein, DE; enthält insges. ca. 0,5 % Emulgator)
- Hypermer LP-1: Oberflächenmodifizierungssmittel auf Basis eines mittelkettigen Polyesters (Uniqema, GB.)
- PPG700DA: Polypropylenglykol-700-diacrylat
- 5-DAMS: Pentaerythritdiacrylat-Monostearat
- TEMPO: 2,2,6,6-Tetramethylpiperidinyloxyl
- Photoinitiator: Bis-(4-methoxybenzoyl)diethylgermanium

### Herstellung des Schlickers:

Das Wachs, die Monomere PPG700DA und 5-DAMS, und das Oberflächenmodifizierungsmittel wurden bei 70°C in einem Dissolver (Dispermat®, Fa. VMA-Getzmann GmbH, Reichshof, DE) vorgelegt. Bei niedrigen Drehzahlen wurde das ZrO₂-Pulver portionsweise zugegeben. Nachdem alles Pulver eingearbeitet wurde, wurde die Drehzahl auf 20.000 min⁻¹ hochgefahren und es wurde mindestens 30 min gemischt. Die aufgrund der hohen Scherkräfte im System auftretende Erwärmung machte eine externe Heizung dabei unnötig, u.U. ist sogar eine Kühlung nötig. Nach den 30 min intensiven Rührens wurde der Schlicker bei leichtem Rühren abgekühlt. Dabei wurden, solange der Schlicker noch flüssig war, Octadecen und TEMPO zugegeben sowie zum Schluss der Photoinitiator.

### Beispiel 3

### Herstellung von Formkörpern

Die hergestellten Schlicker wurde in einem selbstgebauten Digital Light Processing (DLP) Stereolithographiegerät (Blueprinter, TU Wien) zu zylindrischen Prüfkörpern mit einem Durchmesser von 6 mm und einer Höhe von 6 mm verbaut. Die Schlickertemperatur wurde auf ca. 60°C eingestellt. Wichtig ist, dass der Schlicker und alle Komponenten (besonders die Komponenten, die mit der zu bauenden Schicht in Kontakt kommen) auf eine Temperatur oberhalb des Schmelzpunktes der Komponente (d) erwärmt werden. Die Wellenlänge des Lichtes war 460 nm und die Schlicker wurden mit 150mJ/cm² pro Schicht belichtet. Der Abstand zwischen den Schichten wurde auf 25 µm eingestellt.

Nach dem Bauen wurden die Teile manuell von der Bauplatform entfernt, in einer wässrigen Geschirrspülmittellösung bei 50°C für 5-10 Minuten im Ultraschallbad gereinigt und danach trockengeblasen. Die Temperatur der Reinigungslösung lag oberhalb des Schmelzpunktes des der Komponente (d).

Nach dem Trocknen wurden die gereinigten Bauteile in einem Laborofen (Nabertherm GmbH, Deutschland) entbindert. Das verwendete Entbinderungsprogramm für Zylinder mit 6 mm Durchmesser und 6 mm Höhe war: (i) Aufheizen von Raumtemperatur auf 100°C in 2h 40min; (ii) weiteres Aufheizen auf 200°C in 6h 40min; (iii) weiteres Aufheizen auf 420°C dann in 7h 20min; (iv) Halten bei 420°C für 1h. Bei grösseren und dickeren Bauteilen sind entsprechende Anpassungen notwendig. Während des gesamten Entbinderungsvorgangs muss für eine gute Luftzirkulation im Ofenraum gesorgt werden, damit die entstehenden Gase gut abtransportiert werden können.

Zu Beginn des Entbinderns entstanden durch das Verdampfen des Lösungsmittels und das Ausfließen des nichtionischen Tensids Poren. Bei Schlicker 1 war primär das nichtionische Tensid (d) für die Entstehung der Porosität verantwortlich. Diese Poren erleichterten das Entweichen der entstehenden Gase und es wurden defektfrei Weißlinge erhalten, die ohne Probleme gesintert werden konnten.

Danach waren die Grünkörper vollständig entbindert und konnten in einem Sinterofen (Programat P510, Fa. Ivoclar Vivadent) standartmässig dichtgesintert werden. Das Sinterprogramm war: 10K/min bis 700°C, 30min Haltezeit bei 700°C, 10K/min bis 850°C, Vakuum ab 500°C, 1min halten bei 850°C, Abkühlen mit (10K/min) bis 700°C, ca. 90 min.

Die Photopolymerisation des Schlickers aus Beispiel 2 verlief im Stereolithographie-Prozess aufgrund der Reaktionsträgheit der langkettigen Monomere langsam und ergab einen mechanisch wenig stabilen Grünkörper, der äußerst vorsichtige Handhabung erforderte. Auch nach dem Entbindern waren die gedruckten Objekte sehr fragil und empfindlich.

## Patentansprüche

1. Schlicker zur stereolithographischen Herstellung von Keramik- oder Glaskeramikformteilen, der
(a) mindestens ein radikalisch polymerisierbares Monomer,
(b) mindestens einen Photoinitiator und
(c) Keramik- und/oder Glaskeramikpartikel enthält,
**dadurch gekennzeichnet, dass** er
(d) mindestens ein nichtionisches Tensid mit einem Schmelzpunkt von 30°C bis 120 °C enthält.

2. Schlicker nach Anspruch 1, der als nichtionisches Tensid (d) ein Tensid mit einem Schmelzpunkt von 40°C bis 100°C, bevorzugt 40°C bis 60°C enthält.

3. Schlicker nach Anspruch 1 oder 2, der als nichtionisches Tensid ein Tensid mit einem HLB-Wert von 3 bis 16, insbesondere 4 bis 13 und ganz besonders von 4 bis 10 enthält.

4. Schlicker nach einem der Ansprüche 1 bis 3, der als nichtionisches Tensid (d) ein Ethoxylat von Fettalkoholen, Oxoalkoholen oder Fettsäuren, einen Fettsäureester von Zuckern oder hydrierten Zuckern, ein Alkylglycosid, ein Blockpolymer des Ethylen- und Propylenoxids, ein kurzkettiges Block-Co-Oligomer oder eine Mischung davon enthält.

5. Schlicker nach Anspruch 4, der als nichtionisches Tensid (d) einen Fettsäuresester von hydrierten Zuckern mit der Formel R'-CO-O-Zucker enthält, in der R' ein verzweigter oder gerader Alkylrest mit 10 bis 25 Kohlenstoffatomen ist und "Zucker" für einen 1- bis 5-fach ethoxylierten oder nicht ethoxylierten, hydrierten Zuckerrest steht.

6. Schlicker nach Anspruch 4 oder 5, der als nichtionisches Tensid (d) ein Fettsäureethoxylat der allgemeinen Formel R"-(CO)-(OCH₂CH₂)ₘ-OH enthält, in der R" ein verzweigter oder gerader Alkylrest mit 10 bis 25 Kohlenstoffatomen und m ist eine ganze Zahl von 2 bis 20 ist.

7. Schlicker nach einem der Ansprüche 4 bis 6, der als nichtionisches Tensid (d) einen Polyalkylenglycolether mit der allgemeinen Formel R-(OCH₂CH₂)ₙ-OH enthält, in der R ein Alkylrest mit 10 bis 20 Kohlenstoffatomen und n eine ganze Zahl von 2 bis 25 ist.

8. Schlicker nach einem der Ansprüche 1 bis 7, der als Monomer (a) mindestens ein (Meth)acrylat und/oder (Meth)-acrylamid enthält.

9. Schlicker nach einem der Ansprüche 1 bis 8, der als Komponente (c) Keramikpartikel auf der Basis von ZrO₂, Al₂O₃ oder ZrO₂-Al₂O₃ enthält, oder auf der Basis von ZrO₂, Al₂O₃, ZrO₂-Al₂O₃, das jeweils mit HfO₂, CaO, Y₂O₃, CeO₂ und/oder MgO stabilisiert ist.

10. Schlicker nach einem der Ansprüche 1 bis 8, der als Komponente (c) Glaskeramikpartikel auf der Basis von Leucit-, Apatit- und/oder Lithiumdisilikatglaskeramik enthält.

11. Schlicker nach einem der Ansprüche 1 bis 10, bei dem die Partikel der Komponente (c) eine Partikelgröße im Bereich von 10 nm bis 100 µm aufweisen.

12. Schlicker nach einem der Ansprüche 1 bis 11, der zusätzlich mindestens ein Oberflächenmodifizierungsmittel und/oder mindestens ein Additiv enthält, das aus Farbstoffen, Lösungsmitteln, Inhibitoren, Entbinderungsbeschleunigern, Entschäumungsmitteln und/oder Hautverhinderungsmitteln ausgewählt ist.

13. Schlicker nach Anspruch 12, bei dem
- das Oberflächenmodifizierungsmittel aus linearen oder verzweigten Carbonsäuren, Ameisensäure, Essigsäure, Propionsäure, Octansäure, Isobuttersäure, Isovaleriansäure, Pivalinsäure, Phosphonsäuren, Methyl-, Ethyl-, Propyl-, Butyl- Hexyl, Octyl- oder Phenylphosphonsäure, Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan, sauren Phosphorsäureestern, Dimethyl-, Diethyl-, Dipropyl-, Dibutyl-, Dipentyl-, Dihexyl-, Dioctyl- oder Di(2-ethylhexyl)phosphat ausgewählt ist; und/oder
- der Farbstoff aus organischen Farbstoffen ausgewählt ist, vorzugsweise aus Azofarbstoffen, Carbonylfarbstoffen, Cyaninfarbstoffen, Azomethin, Methin, Phthalocyanin, Dioxazin, (4-(4-Nitrophenylazo)anilin oder einer Mischung davon; und/oder
- das Lösungsmittel aus Polyethylenglykolen, Polypropylenglykolen, PEG-PPG-Co-Polymeren, Glycerin und Glycerinderivaten, Phthalaten, PEG 200-600g/mol, PPG 200-800g/mol, co-PEG-PPG 200-800g/mol und Polypropyleneglycol ∼400g/mol und Mischungen davon ausgewählt ist.

14. Schlicker nach einem der Ansprüche 1 bis 13, der
- 5 bis 65 Gew.-%, vorzugsweise 9 bis 57 Gew.-%, besonders bevorzugt 10 bis 40 Gew.-% Monomer (a);
- 0,001 bis 1,0 Gew.-%, vorzugsweise 0,01 bis 1,0 Gew.-%, besonders bevorzugt 0,1 bis 1,0 Gew.-% Photoinitiator (b);
- 33 bis 90 Gew.-%, vorzugsweise 40 bis 88 Gew.-%, besonders bevorzugt 56 bis 86 Gew.-% Keramik- und/oder Glaskeramikpartikel (c);
- 1 bis 30 Gew.-%, vorzugsweise 2 bis 15 Gew.-%, besonders bevorzugt 3 bis 10 Gew.-% nichtionisches Tensid (d) enthält.

15. Verwendung eines Schlickers gemäß einem der Ansprüche 1 bis 14 zur Herstellung von Keramik- oder Glaskeramikformteilen, vorzugsweise einer Dentalrestauration, wie einem Inlay, Onlay, Veneer, einer Krone, Brücke oder eines Gerüsts.

16. Verfahren zur Herstellung eines Keramik- oder Glaskeramikformteils bei dem man
(A) einen Grünling herstellt, indem man einen Schlicker gemäß einem der Ansprüche 1 bis 13 durch lokales Einbringen von Strahlungsenergie unter Ausbildung der geometrischen Form des Grünlings aushärtet,
(B) man dann den Grünling einer Wärmebehandlung zur Entfernung des Bindemittels (Entbinderung) unterwirft, um einen Weissling zu erhalten, und
(C) man den Weissling sintert.

## Claims

1. Slip for the stereolithographic preparation of ceramic or glass ceramic shaped parts, wherein said slip comprises
(a) at least one radically polymerisable monomer,
(b) at least one photoinitiator and
(c) ceramic and/or glass ceramic particles,
**characterised in that** said slip comprises
(d) at least one non-ionic surfactant with a melting point of from 30 °C to 120 °C.

2. Slip according to claim 1, comprising a surfactant as the non-ionic surfactant (d) with a melting point of from 40 °C to 100 °C, preferably 40 °C to 60 °C.

3. Slip according to claim 1 or 2, comprising a surfactant as the non-ionic surfactant with an HLB value of from 3 to 16, in particular 4 to 13 and quite particularly of from 4 to 10.

4. Slip according to any one of claims 1 to 3, comprising as the non-ionic surfactant (d) an ethoxylate of fatty alcohols, oxo alcohols or fatty acids, a fatty acid ester of sugars or hydrogenated sugars, an alkylglycoside, a block polymer of ethylene and propylene oxide, a short-chain block co-oligomer or a mixture thereof.

5. Slip according to claim 4, comprising as the non-ionic surfactant (d) a fatty acid ester of hydrogenated sugars with the formula R'-CO-O-sugar, in which R' is a branched or straight-chain alkyl group having 10 to 25 carbon atoms and "sugar" means a 1 to 5 times ethoxylated or non-ethoxylated hydrogenated sugar residue.

6. Slip according to claim 4 or 5, comprising as the non-ionic surfactant (d) a fatty acid ethoxylate of the general formula R"-(CO)-(OCH₂CH₂)ₘ-OH, in which R" is a branched or linear alkyl group having 10 to 25 carbon atoms and m is an integer from 2 to 20.

7. Slip according to any one of claims 4 to 6, comprising as the non-ionic surfactant (d) a polyalkylene glycol ether with the general formula R-(OCH₂CH₂)ₙ-OH in which R is an alkyl group having 10 to 20 carbon atoms and n is an integer from 2 to 25.

8. Slip according to any one of claims 1 to 7, comprising as the monomer (a) at least one (meth)acrylate and/or (meth)acrylamide.

9. Slip according to one of claims 1 to 8, comprising as the component (c) ceramic particles based on ZrO₂, Al₂O₃ or ZrO₂-Al₂O₃, or based on ZrO₂, Al₂O₃, ZrO₂-Al₂O₃ and stabilized in each case with HfO₂, CaO, Y₂O₃, CeO₂ and/or MgO.

10. Slip according to one of claims 1 to 8, comprising as the component (c) glass ceramic particles based on leucite, apatite and/or lithium disilicate glass ceramic.

11. Slip according to any one of claims 1 to 10, wherein particle size of the particles of component (c) are in the range of from 10 nm to 100 µm.

12. Slip according to one of claims 1 to 11, additionally comprising at least one surface modification agent and/or at least one additive that is selected from dyes, solvents, inhibitors, debinding accelerators, defoaming agents and/or anti-skinning agents.

13. Slip according to claim 12, in which
- the surface modification agent is selected from linear or branched carboxylic acids, formic acid, acetic acid, propionic acid, octanoic acid, *iso*butyric acid, *iso*valeric acid, pivalic acid, phosphonic acids, methyl, ethyl, propyl, butyl, hexyl, octyl or phenylphosphonic acid, propyltrimethoxysilane, phenyltrimethoxysilane, hexyltrimethoxysilane, octyltrimethoxysilane, trimethylchlorosilane, trimethylbromosilane, trimethylmethoxysilane or hexamethyldisilazane, acidic phosphoric acid esters, dimethyl, diethyl, dipropyl, dibutyl, dipentyl, dihexyl, dioctyl or di(2-ethylhexyl) phosphate; and/or
- the dye is selected from organic dyes, preferably from azo dyes, carbonyl dyes, cyanine dyes, azomethine, methine, phthalocyanine, dioxazine, (4-(4-nitrophenylazo)aniline or a mixture thereof; and/or
- the solvent is selected from polyethylene glycols, polypropylene glycols, PEG-PPG co-polymers, glycerol and glycerol derivatives, phthalates, PEG 200-600 g/mol, PPG 200-800 g/mol, co-PEG-PPG 200-800 g/mol and polypropylene glycol ∼400 g/mol and mixtures thereof.

14. Slip according to one of claims 1 to 13 comprising
- 5 to 65 wt %, preferably 9 to 57 wt %, particularly preferably 10 to 40 wt % monomer (a);
- 0.001 to 1.0 wt %, preferably 0.01 to 1.0 wt %, particularly preferably 0.1 to 1.0 wt % photoinitiator (b);
- 33 to 90 wt %, preferably 40 to 88 wt %, particularly preferably 56 to 86 wt % ceramic and/or glass ceramic particles (c);
- 1 to 30 wt %, preferably 2 to 15 wt %, particularly preferably 3 to 10 wt % non-ionic surfactant (d).

15. Use of a slip according to one of claims 1 to 14 for the preparation of ceramic or glass ceramic shaped parts, preferably of a dental restoration, such as an inlay, onlay, veneer, crown, bridge or framework.

16. Process for the preparation of a ceramic or glass ceramic shaped part, in which process
(A) a green compact is prepared by curing a slip according to any one of claims 1 to 13 by locally introducing radiation energy to form the geometric shape of the green compact,
(B) the green compact is then subjected to a heat treatment to remove the binder (debinding), in order to obtain a white body, and
(C) the white body is sintered.

## Revendications

1. Barbotine pour fabrication par stéréolithographie de pièces façonnées en céramique ou en vitrocéramique, qui contient :
a) au moins un monomère polymérisable par voie radicalaire,
b) au moins un photoamorceur,
c) et une céramique ou une vitrocéramique en particules,
**caractérisée en ce qu'**elle contient
d) au moins un tensioactif non-ionique présentant un point de fusion de 30 °C à 120 °C.

2. Barbotine conforme à la revendication 1, qui contient, en tant que tensioactif non-ionique (d), un tensioactif présentant un point de fusion de 40 °C à 100 °C, et de préférence de 40 °C à 60 °C.

3. Barbotine conforme à la revendication 1 ou 2, qui contient, en tant que tensioactif non-ionique, un tensioactif présentant un RHL (rapport hydro-lipophile) de 3 à 16, en particulier de 4 à 13 et surtout de 4 à 10.

4. Barbotine conforme à l'une des revendications 1 à 3, qui contient, en tant que tensioactif non-ionique (d), un produit d'éthoxy-lation d'alcools gras, d'oxo-alcools ou d'acides gras, un ester d'acide gras et d'ose ou d'ose hydrogéné, un alkyl-glycoside, un copolymère à blocs d'oxyde d'éthylène et d'oxyde de propylène, un co-oligomère à blocs à chaîne courte, ou un mélange de tels composés.

5. Barbotine conforme à la revendication 4, qui contient, en tant que tensioactif non-ionique (d), un ester d'acide gras et d'ose hydrogéné, de formule R'-CO-O-ose dans laquelle R' représente un groupe alkyle à chaîne linéaire ou ramifiée comportant de 10 à 25 atomes de carbone et « ose » représente un résidu d'ose hydrogéné, éthoxylé avec 1 à 5 motifs ou non éthoxylé.

6. Barbotine conforme à la revendication 4 ou 5, qui contient, en tant que tensioactif non-ionique (d), un produit d'éthoxylation d'acide gras, de formule générale R"-(CO)-(OCH₂CH₂)ₘ-OH dans laquelle R" représente un groupe alkyle à chaîne linéaire ou ramifiée comportant de 10 à 25 atomes de carbone et l'indice m est un nombre entier valant de 2 à 20.

7. Barbotine conforme à l'une des revendications 4 à 6, qui contient, en tant que tensioactif non-ionique (d), un éther de polyalky-lèneglycol de formule générale R-(OCH₂CH₂)ₙ-OH dans laquelle R représente un groupe alkyle comportant de 10 à 20 atomes de carbone et l'indice n est un nombre entier valant de 2 à 25.

8. Barbotine conforme à l'une des revendications 1 à 7, qui contient, en tant que monomère (a), un (méth)acrylate et/ou un (méth)-acrylamide.

9. Barbotine conforme à l'une des revendications 1 à 8, qui contient, en tant que composant (c), des particules de céramique à base de ZrO₂, Al₂O₃ ou ZrO₂-Al₂O₃, ou de céramique à base de ZrO₂, Al₂O₃ ou ZrO₂-Al₂O₃ stabilisée avec HfO₂, CaO, Y₂O₃, CeO₂ et/ou MgO.

10. Barbotine conforme à l'une des revendications 1 à 8, qui contient, en tant que composant (c), des particules de vitrocéramique à base de leucite, d'apatite et/ou de vitrocéramique de disilicate de lithium.

11. Barbotine conforme à l'une des revendications 1 à 10, dans laquelle les particules du composant (c) ont une taille située dans l'intervalle allant de 10 nm à 100 µm.

12. Barbotine conforme à l'une des revendications 1 à 11, qui contient en outre au moins un agent de modification de surface et/ou au moins un adjuvant choisi parmi les colorants, solvants, inhibiteurs, agents accélérant l'élimination des liants, agents de démoussage et/ou agents anti-peau.

13. Barbotine conforme à la revendication 12, dans laquelle
- l'agent de modification de surface est choisi parmi les suivants : acides carboxyliques à chaîne linéaire ou ramifiée, acide formique, acide acétique, acide propionique, acide octanoïque, acide isobuty-rique, acide isovalérique, acide pivalique, acides phosphoniques, acide méthyl-phosphonique, éthyl-phosphonique, propyl-phosphonique, butyl-phosphonique, hexyl-phosphonique, octyl-phosphonique ou phényl-phosphonique, propyl-triméthoxy-silane, phényl-triméthoxy-silane, hexyl-triméthoxy-silane, octyl-triméthoxy-silane, triméthyl-chloro-silane, triméthyl-bromo-silane, triméthyl-méthoxy-silane ou hexaméthyl-disilazane, esters acides d'acide phosphorique, phosphate de diméthyle, de diéthyle, de dipropyle, de dibutyle, de dipentyle, de dihexyle, de dioctyle ou de di(2-éthyl-hexyle) ;
- et/ou le colorant est choisi parmi les colorants organiques, et de préférence parmi les suivants : colorants azoïques, carbonylés, cyanines, azométhiniques, méthiniques, phtalocyanines, dioxazines, 4-(4-nitro-phényl-azo)-aniline, et les mélanges de tels composés ;
- et/ou le solvant est choisi parmi les suivants : polyéthylène-glycols, polypropylène-glycols, copolymères PEG-PPG, glycérol et dérivés de glycérol, phtalates, PEG de 200 à 600 g/mol, PPG de 200 à 800 g/mol, co-PEG-PPG de 200 à 800 g/mol, polypropylène-glycol d'à peu près 400 g/mol, et les mélanges de tels composés.

14. Barbotine conforme à l'une des revendications 1 à 13, qui contient
- de 5 à 65 % en poids, de préférence de 9 à 57 % en poids, et mieux encore de 10 à 40 % en poids de monomère (a),
- de 0,001 à 1,0 % en poids, de préférence de 0,01 à 1,0 % en poids, et mieux encore de 0,1 à 1,0 % en poids de photoamorceur (b),
- de 33 à 90 % en poids, de préférence de 40 à 88 % en poids, et mieux encore de 56 à 86 % en poids de céramique ou de vitrocéramique en particules (c),
- de 1 à 30 % en poids, de préférence de 2 à 15 % en poids, et mieux encore de 3 à 10 % en poids de tensioactif non-ionique (d).

15. Utilisation d'une barbotine conforme à l'une des revendications 1 à 14, pour la fabrication de pièces façonnées en céramique ou en vitrocéramique, et de préférence, de pièces de restauration dentaire comme une incrustation inlay ou onlay, une facette « veneer », une couronne, un bridge ou une structure de support.

16. Procédé de fabrication d'une pièce façonnée en céramique ou en vitrocéramique, dans lequel
A) on prépare un corps cru en faisant durcir une barbotine, conforme à l'une des revendications 1 à 13, par apport local d'énergie de rayonnement, tout en façonnant le corps cru pour lui donner sa forme géométrique,
B) on soumet ensuite le corps cru à un traitement thermique pour éliminer le liant et obtenir ainsi un corps blanc,
C) et l'on fritte ce corps blanc.
